# EUROPEAN PATENT SPECIFICATION

(11) **EP 1 686 938 B1**
(45) Date of publication and mention of the grant of the patent: **05.08.2015**
(21) Application number: 04811928.3
(22) Date of filing: 22.11.2004
(51) Int. Cl.: A61F 13/475, A61F 13/15, A61F 13/494

(54) **ABSORBENT ARTICLE WITH ELASTICIZED BARRIER CUFFS**
SAUGFÄHIGER ARTIKEL MIT ELASTISCHEN BARRIEREMANSCHETTEN
ARTICLE ABSORBANT AVEC BANDES ELASTIQUES FAISANT BARRIERE

(30) Priority: 21.11.2003 US 524254 P
(43) Date of publication of application: 09.08.2006
(73) Proprietor: THE PROCTER & GAMBLE COMPANY, Cincinnati, OH 45202 (US)
(72) Inventor: SUZUKI, Minako, Kobe (JP); IWATA, Toshiyuki, Nishi-ku, Hyogo 651-2277 (JP)
(74) Representative: Heide, Ute
(86) International application number: PCT/US2004/039296
(87) International publication number: WO 2005/051265

(56) References cited:
- EP-A- 1 106 154
- EP-A- 1 153 587
- US-A- 5 569 234
- US-A- 5 672 166
- US-A- 5 752 947
- US-A1- 2002 058 920

## Description

### FIELD OF THE INVENTION

The present invention relates to absorbent articles used for incontinent babies, children or adults. More particularly, the present invention relates to absorbent articles such as disposable diapers, sanitary napkins, supplemental inner pads used with disposable diapers, and the like, comprising a pair of elasticized barrier cuffs.

### BACKGROUND OF THE INVENTION

Absorbent articles worn to assist in the collection of bodily discharges of incontinent persons are well known in the art. Such conventional arts include disposable diapers, training pants, adult incontinent garments, sanitary napkins, supplemental inner pads used with disposable diapers, and the like. Conventional absorbent articles typically comprise a liquid-permeable topsheet, a liquid impermeable backsheet and an absorbent core positioned between the topsheet and the backsheet. In order to improve the ability of an absorbent article to absorb and reduce leakage of discharged excreta, it has become common to include elastic waistbands and leg elastics on such articles. Some conventional absorbent articles have also included elasticized barrier cuffs at the leg sections to further reduce leakage.

Conventional absorbent articles that include barrier cuffs have not been completely satisfactory. Conventional barrier cuffs in such articles typically include a single cuff positioned at each of sides of the article. Examples of single barrier cuffs are disclosed in Japanese Patent Laid-Open publications 235501/1988, 280951/1991 and 184622/1993. Such a single barrier cuff has a proximal edge attached to the absorbent article and an elasticized distal edge opposite to the proximal edge. Since the single barrier cuff is attached to the article such that the distal edge of the cuff is maintained in a generally upright position to contact with the body of a wearer, it provides a seal that attempts to prevent the flow of bodily exudates. However, a single barrier cuff often fails to provide a sufficient seal between the distal edge of the cuff and the wearer's body. The lack of a sufficient seal results in leakage of exudates, especially runny fecal matter. Moreover, the single barrier cuff is not particularly effective enough to resist forceful and repetitive excretion of bodily exudates. The leakage of exudates past the barrier cuff results in soiling the clothing, bedding, and person.

Accordingly, further attempts have been made to improve the ability of barrier cuffs to prevent leakage of discharged excreta. One of such attempts includes a double barrier cuff structure disclosed in e.g. Japanese Patent Laid-Open publications 218159/1992, 277091/1998, 19121/1999, 123207/1999 and 206810/1999. These patent publications disclose an absorbent article comprising a pair of laterally opposite outer cuffs and a pair of laterally opposite inner cuffs. The combination of one outer cuff with one inner cuff at each of sides of the article functions as a double barrier cuff. Because such a double cuff structure provides a double seal to prevent the flow of bodily exudates, the ability to prevent leakage of discharged excreta is enhanced in comparison with singe barrier cuffs. However, such a double barrier cuff still suffers from the drawback that the distal edge of each cuff hardly follows the movement of the wearer's body in order to continue to contact with the wearer's body during use of the article. The separation of the distal edge of the barrier cuff from the wearer's body can results in leakage of discharged excreta.

As mentioned above, conventional absorbent article with barrier cuffs around the leg openings have not been completely satisfactory. Accordingly, there still exists a need for absorbent article comprising barrier cuffs capable of providing a double seal to effectively prevent the flow of bodily exudates during use of the article.

European Patent Application EP 1 106 154 A2 discloses a disposable sanitary garment which includes a chassis defining an outer surface thereof. The chassis is provided along its transversely opposite side edge portions destined to extend around a wearer's thighs with a pair of first leg-hole elastic members extending circumferentially around the thighs and with a pair of second leg-hole elastic members describing a circular arc being convex inwardly of the garment. The chassis is folded back inwardly of the garment along one side edges of the respective members with the second leg-hole elastic members inside and a portion of the chassis extending inside the zone along which the chassis has been first folded back is folded back along the other side edges of the respective members.

### SUMMARY OF THE INVENTION

An absorbent article (100) has a longitudinal centerline (L) and a transverse centerline (T). The absorbent article (100) comprises an absorbent assembly (200) comprising a liquid pervious topsheet (210), a liquid impervious backsheet (220), and an absorbent core (230) positioned between the topsheet (210) and the backsheet (220); and a pair of barrier cuffs (300) disposed on the absorbent assembly (200) and extending longitudinally. Each barrier cuff (300) has a fixed edge (330) attached to the absorbent assembly (200), a free edge (340) opposite to the fixed edge (330). Each barrier cuff (300) has a first folded portion (350) formed between the fixed edge (330) and the free edge (340) by folding the barrier cuff (300) transversely outwardly at both the longitudinal ends (301, 302) of the barrier cuff (300). Each barrier cuff (300) has a second folded portion (360) formed by folding the barrier cuff (300) transversely outwardly at both the longitudinal ends (301, 302) of the barrier cuff (300) so that the second folded portion (360) is positioned transversely inwardly from the first folded portion (350) and the free edge (340) at each longitudinal end (301, 302) of the barrier cuff (300). Each barrier cuff (300) is provided with at least two elastic members (321, 322) so that at least one elastic member (321) is positioned along the free edge (340) and so that at least one elastic member (322) is positioned along the first folded portion (350).

### BRIEF DESCRIPTION OF THE DRAWINGS

While the Specification concludes with claims which particularly point out and distinctly claim the invention, it is believed the present invention will be better understood from the following description of preferred embodiments taken in conjunction with the accompanying drawings, in which like reference numerals identify identical elements and wherein:
Figure 1 is a top plan view of one embodiment of an absorbent article of the present invention;
Figure 2 is a cross-sectional view taken along line II-II in Figure 1;
Figure 3 is a cross-sectional view of taken along line II-II in Figure 1 if the absorbent article illustrated in Figure 1 further comprises an additional apertured sheet and an acquisition layer;
Figure 4 is a first schematic cross-sectional view illustrating a process for folding a barrier cuff transversely outwardly at the longitudinal end of the barrier cuff;
Figure 5 is a second schematic cross-sectional view illustrating a process for folding a barrier cuff transversely outwardly at the longitudinal end of the barrier cuff;
Figure 6 is a third schematic cross-sectional view illustrating a process for folding a barrier cuff transversely outwardly at the longitudinal end of the barrier cuff; and
Figure 7 is a cross-sectional view taken along line VII-VII in Figure 1.

### DETAILED DESCRIPTION OF THE INVENTION

The definitions of several terms are first provided to assist the reader in understanding the present invention.

The term "absorbent articles", as used herein, refers to devices which absorb and contain body exudates, and more specifically, refers to devices which are placed against or in proximity to the body of the wearer to absorb and contain the various exudates discharged from the body. Such devices include disposable diapers, training pants, adult incontinent garments, sanitary napkins, supplemental inner pads used with disposable diapers, and the like.

The term "comprising" and its derivatives, as used herein, are intended to be open ended terms that specify the presence of the stated features, elements, components, groups, integers, and/or steps, but do not exclude the presence of other, unstated features, elements, components, groups, integers, and/or steps. This definition also applies to words of similar meaning, for example, the terms "has" and "include" and their derivatives.

The terms "elastic" and "elastomeric" and their derivatives, as used herein, refer to a property of a material because of which the material tends to recover its original size and shape after removal of a force causing the material to deform.

The terms "excreta" and "bodily discharges", as used herein, are interchangeable, and each include all discharges released from an excretory orifice of a human body, including fecal materials, urine, menses, and the like. The term "excretory orifice", as used herein, refers to an orifice which excreta pass through to discharge the excreta from the human body when excretion occurs. Such an excretory orifice includes urethra, vaginal orifice, anus, and the like.

The term "inward" or "inwardly", as used herein, is intended to refer to a position located more toward the longitudinal or transverse centerline of an absorbent article. For example, the term "transversely inwardly" is intended to refer to a position located more toward the longitudinal centerline of an absorbent article. Similarly, the term "longitudinally inwardly" is intended to refer to a position located more toward the transverse centerline of an absorbent article.

The term "joined" or "joining", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e. one element is essentially part of the other element. This definition also applies to words of similar meaning, for example, the terms "attached", "bonded", "fixed" and their derivatives.

The term "nonwoven", as used herein, refers to fabrics made of fibers held together by interlocking or bonding which are not woven, knitted, felted, or the like. The term "fabric", as used herein, may refer to a nonwoven web, a woven material, or other types of fabrics.

The term "outward" or "outwardly", as used herein, is intended to refer to a position located more remote from the longitudinal or transverse centerline of an absorbent article. For example, the term "transversely outwardly" is intended to refer to a position located more remote from the longitudinal centerline of an absorbent article. Similarly, the term "longitudinally outwardly" is intended to refer to a position located more remote from the transverse centerline of an absorbent article.

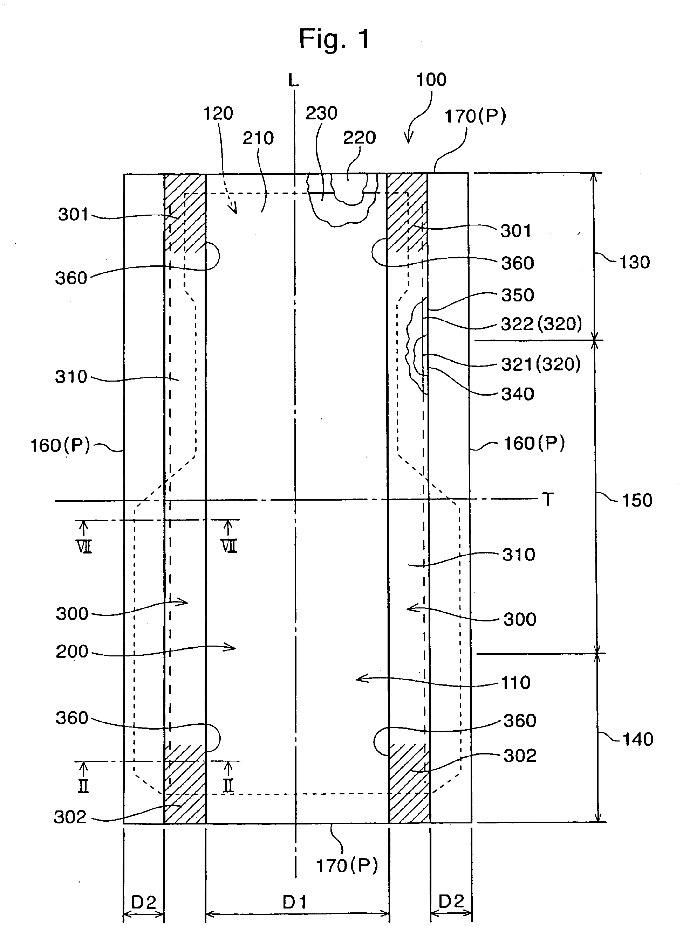

Figures 1, 2 and 4 to 7 illustrate one embodiment of an absorbent article according to the present invention. The absorbent article 100 illustrated in the figures is a supplemental inner pad used with e.g., a conventional disposable diaper. Such a supplemental inner pad is typically utilized by inserting the inner pad between a disposable diaper and the wearer's body during use of the inner pad. Figure 1 illustrates a top plan view of the absorbent article 100 according to the present invention, in its flat-out, uncontracted state (i.e., with elastic-induced contraction being pulled out) with portions of the structure being cut-away to show the underlying features. As illustrated in Figure 1, the absorbent article 100 has a longitudinal centerline L and a transverse centerline T. The term "longitudinal", as used herein, refers to a line, axis or direction in the plane of the absorbent article 100 that is generally aligned with (e.g., approximately parallel to) a vertical plane which bisects a standing wearer into left and right body halves when the absorbent article is worn. The term "transverse" or "lateral", as used herein, are interchangeable, and refer to a line, axis or direction which lies within the plane of the absorbent article 100 that is generally perpendicular to the longitudinal direction. The absorbent article 100 has two surfaces; one is a wearer-facing surface 110 and the other is an opposing surface 120. The wearer-facing surface 110 is the surface of the article 100 which is generally oriented toward the wearer when the article is worn. The wearer-facing surface 110 typically at least partially comes in contact with the wearer's skin during use of the article 100. The opposing surface 120 is the surface of the article 100 which is generally oriented away from the wearer when the article 100 is worn, and at least partially toward a garment if a garment is worn. In Figure 1, the wearer-facing surface 110 of the absorbent article 100 faces the viewer. The absorbent article 100 further has a front region 130, a rear region 140 opposed to the front region 130 with respect to the transverse centerline T, and a crotch region 150 interconnecting the front region to the rear region. During use of the absorbent article 100, the front region 130 abuts the lower front torso of the wearer, the rear region 140 abuts lower rear torso of the wearer, and the crotch region 150 extends from the front region 130 to the rear region 140 through the crotch of the wearer. The absorbent article 100 also has a periphery P which is defined by side edges 160 and end edges 170. The front region 130 and the rear region 140 extend from the end edges 170 toward the crotch region 150. The absorbent article 100 of the present invention comprises an absorbent assembly 200 and a pair of elasticized barrier cuffs 300.

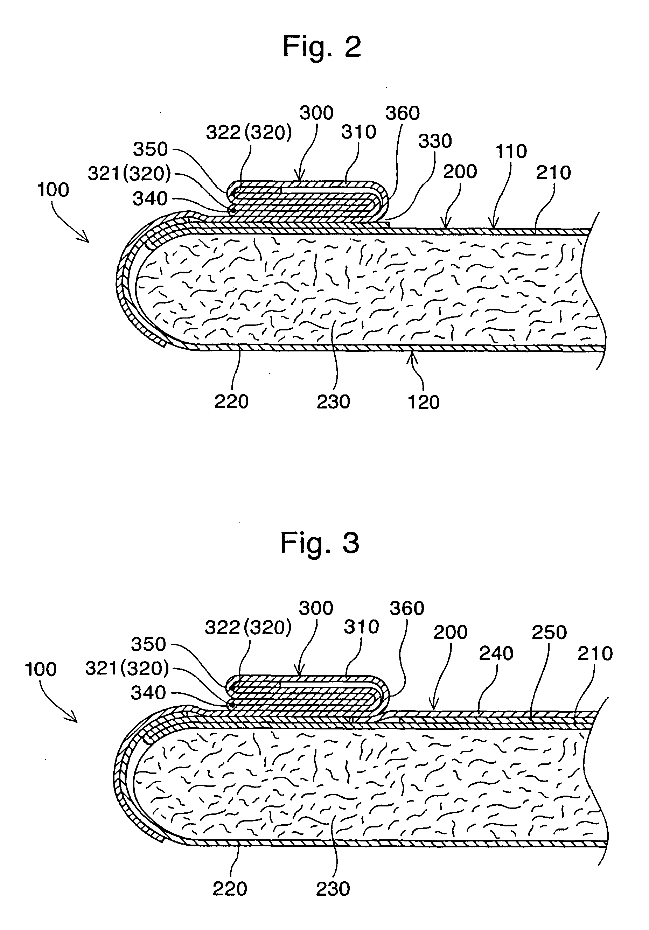

The absorbent assembly 200 typically comprises a liquid pervious topsheet 210, a liquid impervious backsheet 220, and an absorbent core 230 positioned between the topsheet 210 and the backsheet 220 to acquire and store bodily discharges. The topsheet 210 is preferably positioned so as to be adjacent to the wearer-facing surface of the absorbent core 230 and is preferably joined thereto and to the backsheet 220 by any means (not shown) such as those well known in the art. Such means are described below with respect to joining the backsheet 220 to the absorbent core 230. The topsheet 210 and the backsheet 220 may be joined directly to each other. Alternatively, the topsheet 210 and the backsheet 220 may be indirectly joined together by directly joining them to other elements such as the absorbent core 230, the elasticized barrier cuffs 300, and the like by any suitable means. The backsheet 220 is preferably positioned so as to be adjacent to the opposing surface of the absorbent core 230 and is preferably joined thereto by any suitable means known in the art. For example, the backsheet 220 may be secured to the absorbent core 230 by a uniform continuous layer of adhesive, a patterned layer of adhesive, or an array of separate lines, spirals or spots of adhesive. Adhesive which has been found to be satisfactory is, for example, manufactured by H. B. Fuller Company of St. Paul, Minnesota and marketed as HL-1258. Heat bonds, pressure bonds, ultrasonic bonds, dynamic mechanical bonds, or any other suitable means or combinations thereof as are known in the art may be used. While the topsheet 210, the backsheet 220, and the absorbent core 230 may be assembled in a variety of well known configurations in order to form the absorbent assembly 200, exemplary assembly configurations are described generally in U.S. Patent No. 5,074,854; U.S. Patent No. 4,710,187; and International Patent Publication No. WO 90/04375. Such an absorbent assembly may further comprise other features added to form the composite absorbent assembly structure. As illustrated in Figure 3, for example, the absorbent assembly 200 may further comprise an additional apertured sheet 240 as an uppermost layer, and an acquisition nonwoven layer 250 positioned between the apertured sheet 240 and the topsheet 210 in order to allow the absorbent assembly 200 to acquire and distribute liquid bodily discharges more effectively. Such a combination of the apertured topsheet 240 and the acquisition nonwoven layer 250 helps to decelerate and diffuse liquid bodily discharges that may be rapidly introduced into the absorbent core 230 of the absorbent assembly 200. The structure comprising the combination of the apertured topsheet 240 and the acquisition nonwoven layer 250 may also be configured to rapidly accept and temporarily hold liquid bodily discharges prior to releasing the liquid bodily discharges into the absorbent core 230.

The topsheet 210 is adapted to contact the wearer's skin. The topsheet 210 faces the wearer while the absorbent article 100 is worn, and is suitably employed to help isolate the wearer's skin from bodily discharges held in the absorbent core 230. Thus, the topsheet 210 is preferably compliant, soft feeling, and non-irritating to the wearer's skin. Further, the topsheet 210 may be less hydrophilic than the absorbent core 230 to present a relatively dry surface to the wearer. The topsheet 210 may also be liquid pervious for permitting liquid discharges (e.g., urine) to readily penetrate through its thickness. A suitable topsheet may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be composed of natural fibers (e.g., wood or cotton fibers), synthetic fibers (e.g., polymeric fibers such as polyester, polypropylene, polyolefin, or polyethylene fibers), or a combination of natural and synthetic fibers. For example, the topsheet 210 may be composed of a meltblown or spunbonded web of polyolefin fibers. The topsheet 210 may also be a bonded-carded web composed of natural and/or synthetic fibers. Further, the topsheet 210 may be composed of a substantially hydrophobic material, and the hydrophobic material may optionally be treated with a surfactant or otherwise processed to impart a desired level of wettability and hydrophilicity. Such surfactants may be applied by any conventional means, such as spraying, printing, brush coating or the like. Surfactants may be applied to the entire topsheet 210 or may be selectively applied to particular sections of the topsheet 210, such as the medial section along the longitudinal centerline of the absorbent article, to provide greater wettability of such sections. The topsheet 210 may further include a composition applied thereto that is configured to be transferred to the wearer's skin for improving the skin health of the wearer.

The backsheet 220 is to prevent bodily discharges absorbed and contained in the absorbent core 230 from wetting other articles which contact the absorbent article 100 such as bed sheets and undergarments. Thus, the backsheet 220 is preferably impervious to liquids (e.g., urine), and is preferably manufactured from a thin plastic film although other flexible liquid impervious materials may also be used. As used herein, the term "flexible" refers to materials which are compliant and will readily conform to the general shape and contours of the human body. A suitable material for the backsheet 220 is a thermoplastic film having a thickness of from about 0.012 mm (0.5 mil) to about 0.051 mm (2.0 mils), preferably comprising polyethylene or polypropylene. If it is desired to present the backsheet 220 with a more cloth-like feel, the backsheet 220 may comprise a polyolefin film having a nonwoven web laminated to the exterior surface thereof, such as a spunbond web of polyolefin fibers. Further, the backsheet 220 may be formed of a woven or nonwoven fibrous web layer that has been totally or partially constructed or treated to impart a desired level of liquid impermeability to selected regions that are adjacent or proximate to the absorbent core 230. Still further, the backsheet 220 may be optionally composed of a micro-porous "breathable" material that permits vapors to escape from the absorbent article 100 while still preventing liquid discharges form passing through the backsheet 220. For example, the backsheet 220 may include a vapor permeable nonwoven facing layer laminated to a micro-porous film. The backsheet 220 may also be an elastomeric material, such as a stretch-thermal laminate ("STL"), neck-bonded laminate ("NBL"), or stretch-bonded laminate ("SBL") material. The backsheet 220 can also be embossed or otherwise provided with a matte finish to provide a more aesthetically pleasing appearance.

The absorbent core 230 may be any absorbent member which is generally compressible, conformable, non-irritating to the wearer's skin, and capable of absorbing and retaining liquids such as urine and other certain body discharges. The absorbent core 230 may be manufactured in a wide variety of sizes and shapes (e.g., rectangular, hourglass, "T"-shaped, asymmetric, etc.) and from a wide variety of liquid-absorbent materials commonly used in disposable diapers and the other absorbent articles such as comminuted wood pulp which is generally referred to as airfelt. Examples of other suitable absorbent materials include creped cellulose wadding; meltblown polymers including coform; chemically stiffened, modified or cross-linked cellulose fibers; tissue including tissue wraps and tissue laminates; absorbent foams; absorbent sponges; superabsorbent polymers; absorbent gelling materials; or any equivalent material or combinations of materials. Preferably, the absorbent core 230 includes superabsorbent particles and a carrier means for the superabsorbent particles. Such superabsorbent particles are typically manufactured from an absorbent gelling material. Preferably, the carrier means may be formed from comminuted wood pulp. Such comminuted wood pulp may be exchanged with synthetic, polymeric, meltblown fibers or with a combination of meltblown fibers and natural fibers. The configuration and construction of the absorbent core 230 may vary (e.g., the absorbent core may have varying caliper zones, a hydrophilic gradient, a superabsorbent gradient, or lower average density and lower average basis weight acquisition zones; or may comprise one or more layers or structures). Further, the size and absorbent capacity of the absorbent core 230 may also be varied to accommodate wearers ranging from infants through adults. However, the total absorbent capacity of the absorbent core 230 should be compatible with the intended use of the absorbent article 100. The absorbent core 230 may further comprise an acquisition/distribution layer of chemically stiffened fibers and a fluid storage layer positioned underneath the acquisition/distribution layer.

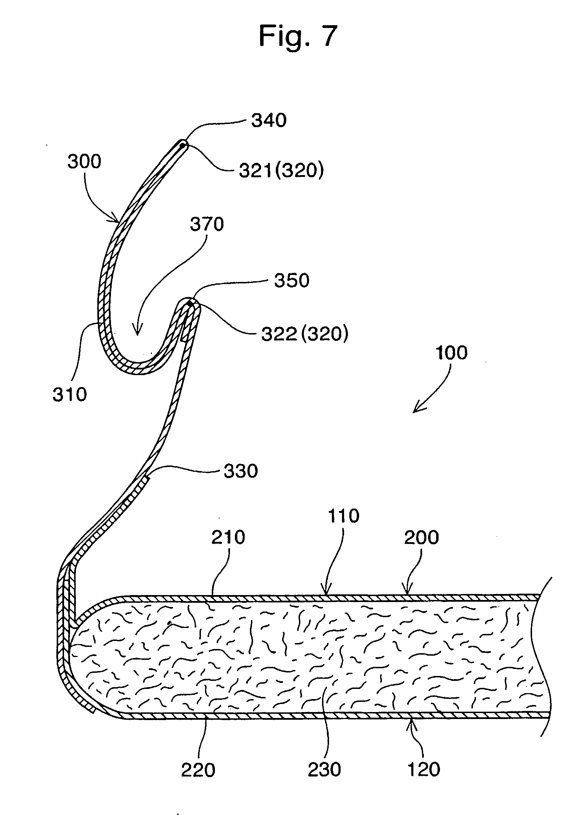

The elasticized barrier cuffs 300 are typically disposed on the wearer-facing surface of the absorbent assembly 200 so that each of the barrier cuffs 300 extends in the longitudinal direction of the absorbent article 100. Such barrier cuffs provide improved containment of liquids and other bodily discharges. Each barrier cuff 300 has a fixed edge 330 attached to the absorbent assembly 200, and a free edge 340 opposite to the fixed edge 330. To maintain a seal between the barrier cuff 300 and the absorbent assembly 200, the fixed edge 330 is joined to the underlying component, such as the topsheet 210, the backsheet 220, the absorbent core 230, or the like, by means (not shown) such as those well known in the art. For example, as representatively illustrated in Figures 2 and 7, the fixed edge 330 of each barrier cuff 300 may be joined to the topsheet 210 at least in the crotch region 150 and preferably along the entire length of the barrier cuff 300. In another preferable embodiment, the fixed edge 330 may be directly bonded to the wearer-facing surface of the absorbent assembly 200. Thus, the barrier cuff 300 is cantilevered from the absorbent assembly 200 at least in the crotch region 150. At least a portion of the free edge 340 of each barrier cuff 300 is not attached to the absorbent assembly 200 such that the barrier cuff 300 provides a barrier to the lateral flow of discharged excreta. For example, the free edge 340 of each barrier cuff 300 may be joined to the absorbent assembly 200 in at least a portion of the front region 130 and the rear region 140 and remain unjoined to the absorbent assembly 200 in at least a portion of the crotch region 150. In such a configuration, the free edge 340 of each barrier cuff 300 is configured to position itself in a spaced relation away from the absorbent assembly 200 toward a generally upright and approximately perpendicular configuration in at least the crotch region 150. The barrier cuff 300 according to the present invention has a first folded portion 350 and a second folded portion 360. The first folded portion 350 is formed between the fixed edge 330 and the free edge 340 by folding the barrier cuff 300 transversely outwardly once at both the longitudinal ends 301, 302 of the barrier cuff 300. The second folded portion 360 is formed by folding the barrier cuff 300 transversely outwardly once more at both the longitudinal ends 301, 302 of the barrier cuff 300. As a result, the second folded portion 360 is positioned transversely inwardly from the first folded portion 350 and the free edge 340 at each longitudinal end 301, 302 of the barrier cuff 300.

Each of the barrier cuffs 300 preferably comprises a barrier sheet 310 and at least one elastic member 320 to elasticize the barrier cuff 300. The elastic member 320 is preferably enclosed by the barrier sheet 310 such that the elastic member 320 is secured to a selected location in the barrier cuff 300. The elastic member 320 allows the barrier cuff 300 to stand up from the absorbent assembly 200 at least in the crotch region 150 of the absorbent article 100. To achieve such a substantially upright configuration, each barrier cuff 300 includes at least one elastic member 320 along at least a portion of the free edge 340 of the barrier cuff 300, preferably, along the entire free edge 340 of the barrier cuff 300. The elastic member 320 is secured to the barrier sheet 310 in an elastically contractible condition. For example, the elastic member 320 may be elongated and secured to the barrier sheet 310 while the barrier cuff 300 is in an uncontracted condition. As a result, the free edge 340 of each barrier cuff 300 tends to contract or gather and position itself in a spaced relation away from the absorbent assembly 200, in particular, away from the topsheet 210 toward a generally upright and approximately perpendicular configuration. Each barrier cuff 300 includes any number of individual elastic members 320 which provide the desired spaced away configuration. In a preferable embodiment, each barrier cuffs 300 is provided with at least two elastic members 321, 322 so that at least one elastic member 321 is positioned along the free edge 340 and so that at least one elastic member 322 is positioned along the first folded portion 350. The barrier cuff 300 may also be provided with a plurality of elastic members so that two or more elastic members are positioned along each of the free edge 340 and the first folded portion 350. Preferably, the elastic member 320 may be configured parallel to the free edge 340 and/or the first folded portion 350 and be located within about 0.5 centimeters from the free edge 340 or the first folded portion 350.

The barrier sheet 310 may be manufactured from a wide variety of materials. For example, materials suitable for the barrier sheet 310 can include a nonwoven material such as a spunbond, meltblown, spun laced or carded polymeric material; a film material such as a polyolefin, polypropylene, polyester, polyurethane, rayon, or nylon film; a foam material; or combinations thereof. The barrier sheet 310 may also be manufactured from materials described above as being suitable for the topsheet 210 or the backsheet 220. In a preferable embodiment, the barrier sheet 310 may be manufactured from a nonwoven material such as a spunbond or meltblown, polyethylene or polypropylene material. Materials for the barrier sheet 310 can also include nonwoven materials, such as thermoplastic polymers, such as polyolefins; bonded carded webs; film materials, such as ethylene vinyl acetate, and ethyl methacrylate films; foam materials, such as polyolefin foams; woven materials, such as woven polypropylene, polyethylene or polyester fabrics; and composites and laminates of the above nonwoven, film, foam, and woven materials. A preferred barrier sheet may also comprise a polypropylene material containing no finish or surfactant to render it liquid impermeable.

The elastic member 320 (321, 322) preferably comprises any elastomeric material capable of being elongated at least about 50 percent, desirably about 350 percent, and capable of elastically contracting after removal of a force elongating the elastic member. For example, the elastic member 320 can comprise a spandex elastomeric strand such as e.g., a 470-decitex Lycra thread commercially available from E. I. Dupont de Nemours and Co. Alternatively, the elastic member 320 can be composed of a thermoplastic elastomer or a natural or systhetic rubber commercially available from J.P.S. Elastomerics Corp. The elastic member 320 can also be composed of a heat activatable elastic material such as PEBAX, commercially available from Atochem, Inc., which can be activated with heat treatment after the elastic member is secured to the barrier cuff. The elastic member 320 can be attached to the barrier cuff 300 by any means known to those skilled in the art such as thermal bonding, adhesive bonding, ultrasonic bonding or the like.

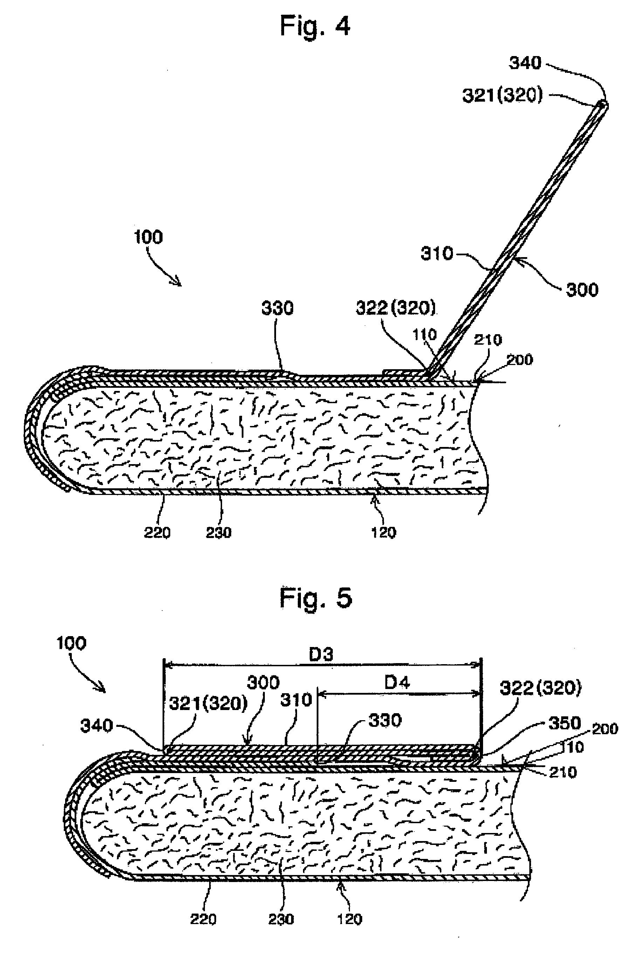

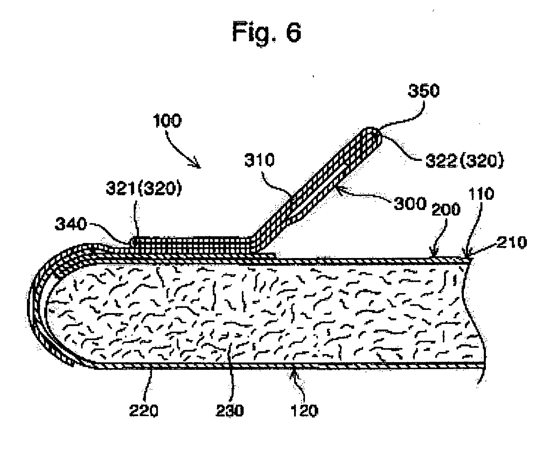

Referring to Figures 2 and 4 to 6, the steps to form the first folded portion 350 and the second folded portion 360 are illustrated. As illustrated in Figure 4, each barrier cuff 300 is folded transversely outwardly at both the longitudinal ends 301, 302 of the barrier cuff 300. As a result, the first folded portion 350 is formed between the fixed edge 330 and the free edge 340 as illustrated in Figure 5. As illustrated in Figure 6, each barrier cuff 300 is further folded transversely outwardly at both the longitudinal ends 301, 302 of the barrier cuff 300. In other words, each barrier cuff 300 is folded transversely outwardly twice in all at both the longitudinal ends 301, 302 of the barrier cuff 300. As a result, the second folded portion 360 is formed so that the second folded portion 360 is positioned transversely inwardly from the first folded portion 350 and the free edge 340 at each longitudinal end 301, 302 of the barrier cuff 300 as illustrated in Figure 2. To maintain its folded configuration shown in Figure 2 at each longitudinal end 301, 302 of the barrier cuff 300, each barrier cuff 300 is then joined to the absorbent assembly 200 at each longitudinal end 301, 302 of the barrier cuff 300 by any means known to those skilled in the art such as thermal bonding, adhesive bonding, ultrasonic bonding or the like.

Folding the barrier cuff 300 transversely outwardly twice in all at each longitudinal end 301, 302 thereof as well as providing the barrier cuff 300 with the elastic members 321 and 322 along the free edge 340 and the first folded portion 350 respectively, allows the barrier cuff 300 to stand up away from the absorbent assembly 200 in the crotch region 150 of the absorbent article 100 in such a manner as illustrated in Figure 7. Because the elastic members 321 and 322 elasticize the free edge 340 and the first folded portion 350 respectively, the barrier cuff 300 forms a dual seal structure in which the elasticized free edge 340 and the elasticized first folded portion 350 function as "an outer seal" and "an inner seal" respectively. Such a dual seal structure also provides a pocket area 370 formed by the barrier sheet 310 between the free edge 340 and the first folded portion 350. Even if discharged excreta pass over the first folded portion 350, such excreta are eventually caught and retained into the pocket area 370. Therefore, the barrier cuff 300 according to the present invention effectively prevents lateral leakage of discharged excreta during use of the absorbent article 100. In addition, because the barrier cuff 300 according to the present invention stands up from the absorbent assembly 200 with the barrier sheet 310 between the free edge 340 and the first folded portion 350 being loose, the free edge 340 elasticized by the elastic member 321 effectively conforms the movement of the skin of a wearer while the absorbent article 100 is worn. For example, when the wearer's skin accidentally comes away from the free edge 340 of the barrier cuff 300 due to wearer's motions such as walking, crawling, running, sitting and the like, the free edge 340 still tends to follow the wearer's skin so that the free edge 340 continues to contact with the wearer's skin. Such dynamic contact of the free edge 340 with the wearer's skin during use advantageously reduces the amount or level of leakage of discharged excreta from the absorbent article 100.

In a preferable embodiment, the barrier cuff 300 may be folded transversely outwardly twice in all at the longitudinal ends 301, 302 of the barrier cuff 300 so that the first folded portion 350 is aligned with the free edge 340 at each longitudinal end 301, 302 of the barrier cuff 300. Such an arrangement allows the free edge 340 to stand up in the crotch region 150 moderately higher than the first folded portion 350. In another preferable embodiment, the barrier cuff 300 may be folded transversely outwardly twice in all at the longitudinal ends 301, 302 of the barrier cuff 300 so that the first folded portion 350 is positioned transversely outwardly from the free edge 340 at each longitudinal end 301, 302 of the barrier cuff 300. Such an arrangement is typically used when it is anticipated that a more height of the first folded portion 350 in the crotch region 150 is needed to enhance the inner seal formed by the first folded portion 350 in the crotch region 150. In yet another preferable embodiment, the barrier cuff 300 may be folded transversely outwardly twice in all at the longitudinal ends 301, 302 of the barrier cuff 300 so that the first folded portion 350 is positioned transversely inwardly from the free edge 340 at each longitudinal end 301, 302 of the barrier cuff 300. Such an arrangement is typically used when it is anticipated that a more height of the free edge 340 in the crotch region 150 is needed to enhance the outer seal formed by the free edge 340 in the crotch region 150.

The elasticized free edge 340 and the elasticized first folded portion 350 of the barrier cuff 300 of the present invention may also define an elasticized length. The term "elasticized length", as used herein, means the total length along the free edge 340 or the first folded portion 350 that may be under tension due to the elastic member 320 within the barrier cuff 300. The elasticized length of the free edge 340 may be substantially equal to that of the first folded portion 350. In a preferable embodiment, the free edge 340 and the first folded portion 350 may each have an elasticized length of about 100% of the overall longitudinal length of the absorbent article 100. In another preferable embodiment, the free edge 340 and the first folded portion 350 may each have elasticized lengths of from about 40% to about 100% of the overall longitudinal length of the absorbent article 100. In yet another preferable embodiment, the free edge 340 and the first folded portion 350 may each have elasticized lengths of from about 60% to about 90% of the overall longitudinal length of the absorbent article 100.

The free edge 340 and the first folded portion 350 may be also configured to have different elasticized lengths. Preferably, the free edge 340 may have a longer elasticized length than the firs folded portion 350. Such an arrangement would advantageously provide improved fit and containment benefits. Specifically, the longer elasticized length of the free edge 340 would allow the free edge 340 to better follow the contour of the wearer's leg in use, while the shorter elasticized length of the first folded portion 350 would provide an inner seal that first stops the lateral flow of discharged excreta in the crotch region 150 of the absorbent article 100. Preferably, the free edge 340 would define an elasticized length of from about 40% to about 100% of the overall longitudinal length of the absorbent article 100 while the first folded portion 350 defines an elasticized length that is less than that of the free edge 340, of from about 20% to about 80% of the overall longitudinal length of the absorbent article 100. More preferably, the free edge 340 would define an elasticized length of from about 65% to about 80% of the overall longitudinal length of the absorbent article 100 while the first folded portion 350 defines an elasticized length of from about 40% to about 60% of the overall longitudinal length of the absorbent article 100. In an advantageous embodiment, the free edge 340 would define an elasticized length of about 75% of the overall longitudinal length of the absorbent article 100 while the first folded portion 350 would define an elasticized length of about 50% of the overall longitudinal length of the absorbent article 100.

Alternatively, the first folded portion 350 may have a longer elasticized length than the free edge 340. The longer elasticized length of the first folded portion 350 effectively reduces the likelihood that the barrier sheet 310 between the free edge 340 and the first folded portion 350 is soiled by discharged excreta because an inner seal formed by the elasticized first folded portion 359 is enhanced. Such an arrangement would advantageously provide a user and/or a wearer with a secure feeling that leakage of excreta is being prevented. Preferably, the first folded portion 350 would define an elasticized length of from about 40% to about 100% of the overall longitudinal length of the absorbent article 100 while the free edge 340 defines an elasticized length that is less than that of the first folded portion 350, of from about 30% to about 95% of the overall longitudinal length of the absorbent article 100. More preferably, the first folded portion 350 would define an elasticized length of from about 80% to about 95% of the overall longitudinal length of the absorbent article 100 while the free edge 340 defines an elasticized length of from about 50% to about 75% of the overall longitudinal length of the absorbent article 100. In an advantageous embodiment, the first folded portion 350 would define an elasticized length of about 80% of the overall longitudinal length of the absorbent article 100 while the free edge 340 would define an elasticized length of about 75% of the overall longitudinal length of the absorbent article 100.

The elasticized length of the free edge 340 and the first folded portion 350 may be provided in any number of means, as appreciated by one of skill in the art. For example, the elastic member 320 may be provided along the free edge 340 (or the first folded portion 350) over only a particular portion thereof. Alternatively, the elastic member 320 may be disposed substantially continuously along the free edge 340 (or the first folded portion 350) over the overall length thereof while the elastic member 320 is configured to be in a relaxed state in certain portions thereof. Specifically, the elastic member 320 may not be actively attached to the barrier sheet 310 in portions of the free edge 340 (or the first folded portion 350). As such, the elastic member 320 would not exhibit tension in those portions where the elastic member 320 was not attached.

The distance D1 between one and the other of two second folded portions 360 oppositely positioned with respect to the longitudinal centerline L of the absorbent article 100 can vary depending on the type and size of the absorbent article 100 to which the barrier cuff 300 will be attached. Larger distances D1 between two second folded portions 360 are typically used when it is anticipated that more area or volume is needed to provide an adequate void volume to entrap discharged excreta. As illustrated in Figure 1, the distance D1 is measured at the longitudinal end 301 (or 302) of the barrier cuff 300 along the transverse direction of the absorbent article 100. The distance D1 may be between about 40 mm and about 250 mm, preferably between about 80 mm and about 150 mm, if the absorbent article 100 is designed for adult wearers. In addition, the distance D1 may be between about 40 mm and about 160 mm, preferably between about 45 mm and about 130 mm, if the absorbent article 100 is designed for infant wearers. It will be appreciated that other distances between two second folded portions 360 are within the scope of the present invention.

The distance D2 between the free edge 340 (or the first folded portion 350) of the barrier cuff 300 and the side edge 160 of the article 100 may vary along the longitudinal direction of the absorbent article 100 since the barrier cuff 300 may not run parallel to the side edge 160 of the article 100 (depending on the shape of the article 100). Typically, the free edge (or the first folded portion) of each barrier cuff can be closest to the side edge of the absorbent article if the article is a disposable diaper. As illustrated in Figure 1, the distance D2 is measured along the transverse direction of the absorbent article 100. The distance D2 may be between about 0 mm and about 140 mm, preferably between about 10 mm and about 90 mm, if the absorbent article 100 is designed for adult wearers. In addition, the distance D2 may be between about 0 mm and about 80 mm, preferably between about 10 mm and about 60 mm, if the absorbent article 100 is designed for infant wearers. Further, the free edge 340 (or the first folded portion 350) of the barrier cuff 300 may be preferably no closer than 5 mm from the side edge 160 of the article 100 at any point along the longitudinal direction. This provides a manufacturing clearance so that the barrier cuff 300 is not damaged when the absorbent article 100 is trimmed in the manufacturing process. It will be appreciated that other distances between the free edge 340 (or the first folded portion 350) of the barrier cuff 300 and the side edge 160 of the article 100 are within the scope of the present invention.

The distance D3 between the free edge 340 and the first folded portion 350 can vary depending on the type and size of the absorbent article 100 to which the barrier cuff 300 will be attached. Larger distances D3 between the free edge 340 and the first folded portion 350 are typically used when it is anticipated that more volume of the pocket area 370 is needed to provide a sufficient void space for prevention of lateral leakage of discharged excreta. As illustrated in Figure 5, the distance D3 is measured at the longitudinal end 301 (or 302) of the barrier cuff 300 along the transverse direction of the absorbent article 100 when the barrier cuff 300 is folded transversely outwardly once at both the longitudinal ends 301, 302 of the barrier cuff 300. The distance D3 may be between about 20 mm and about 120 mm, preferably between about 30 mm and about 80 mm, if the absorbent article 100 is designed for adult wearers. In addition, the distance D3 may be between about 20 mm and about 80 mm, preferably between about 30 mm and about 60 mm, if the absorbent article 100 is designed for infant wearers. It will be appreciated that other distances between the free edge 340 and the first folded portion 350 are within the scope of the present invention.

The distance D4 between the fixed edge 330 and the first folded portion 350 can vary depending on the type and size of the absorbent article 100 to which the barrier cuff 300 will be attached. Larger distances D4 between the fixed edge 330 and the first folded portion 350 are typically used when it is anticipated that more effective height of the first folded portion 350 in at least the crotch region 150 is needed to provide a sufficient barrier height formed by the first folded portion 350 for prevention of lateral leakage of discharged excreta. Further, lager total distances of D3 and D4 are typically used when it is anticipated that more effective height of the barrier cuff 300 in at least the crotch region 150 is needed to prevent lateral leakage of discharged excreta. As illustrated in Figure 5, the distance D4 is measured at the longitudinal end 301 (or 302) of the barrier cuff 300 along the transverse direction of the absorbent article 100 when the barrier cuff 300 is folded transversely outwardly once at both the longitudinal ends 301, 302 of the barrier cuff 300. The distance D4 may be between about 10 mm and about 120 mm, preferably between about 20 mm and about 90 mm, if the absorbent article 100 is designed for adult wearers. In addition, the distance D4 may be between about 10 mm and about 80 mm, preferably between about 20 mm and about 50 mm, if the absorbent article 100 is designed for infant wearers. It will be appreciated that other distances between the fixed edge 330 and the first folded portion 350 are within the scope of the present invention.

## Claims

1. An absorbent article (100) having a longitudinal centerline (L) and a transverse centerline (T), the absorbent article (100) comprising:
an absorbent assembly (200) comprising a liquid pervious topsheet (210), a liquid impervious backsheet (220), and an absorbent core (230) positioned between the topsheet (210) and the backsheet (220); and
a pair of barrier cuffs (300) disposed on the absorbent assembly (200) and extending longitudinally, each barrier cuff (300) having :
a fixed edge (330) attached to the absorbent assembly (200),
a free edge (340) opposite to the fixed edge (330),
a first folded portion (350) formed between the fixed edge (330) and the free edge (340) by folding the barrier cuff (300) transversely outwardly at both the longitudinal ends (301, 302) of the barrier cuff (300), and
a second folded portion (360) formed by folding the barrier cuff (300) transversely outwardly at both the longitudinal ends (301, 302) of the barrier cuff (300) so that the second folded portion (360) is positioned transversely inwardly from the first folded portion (350) and the
free edge (340) at each longitudinal end (301, 302) of the barrier cuff (300),
each barrier cuff (300) being provided with at least two elastic members (321, 322) so that at least one elastic member (321) is positioned along the free edge (340) and so that at least one elastic member (322) is positioned along the first folded portion (350).

2. An absorbent article (100) according to Claim 1 wherein the barrier cuff (300) is folded transversely outwardly at the longitudinal ends (301, 302) of the barrier cuff (300) so that the first folded portion (350) is aligned with the free edge (340) at each longitudinal end (301, 302) of the barrier cuff (300).

3. An absorbent article (100) according to Claim 1 wherein the barrier cuff (300) is folded transversely outwardly at the longitudinal ends (301, 302) of the barrier cuff (300) so that the first folded portion (350) is positioned transversely outwardly from the free edge (340) at each longitudinal end (301, 302) of the barrier cuff (300).

4. An absorbent article (100) according to Claim 1 wherein the barrier cuff (300) is folded transversely outwardly at the longitudinal ends (301, 302) of the barrier cuff (300) so that the first folded portion (350) is positioned transversely inwardly from the free edge (340) at each longitudinal end (301, 302) of the barrier cuff (300).

5. An absorbent article (100) according to Claim 1 wherein the free edge (340) has a longer elasticized length than the first folded portion (350).

6. An absorbent article (100) according to Claim 5 wherein the free edge (340) defines an elasticized length of from about 65% to about 80% of the overall longitudinal length of the absorbent article (100) while the first folded portion (350) defines an elasticized length of from about 40% to about 60% of the overall longitudinal length of the absorbent article (100).

7. An absorbent article (100) according to Claim 1 wherein the first folded portion (350) has a longer elasticized length than the free edge (340).

8. An absorbent article (100) according to Claim 7 wherein the first folded portion (350) defines an elasticized length of from about 80% to about 95% of the overall longitudinal length of the absorbent article (100) while the free edge (340) defines an elasticized length of from about 50% to about 75% of the overall longitudinal length of the absorbent article (100).

## Patentansprüche

1. Absorptionsartikel (100) mit einer Längsachse (L) und einer Querachse (T), wobei der Absorptionsartikel (100) Folgendes umfasst:
eine Absorptionseinheit (200), umfassend eine flüssigkeitsdurchlässige Oberschicht (210), eine flüssigkeitsundurchlässige Unterschicht (220) und einen Absorptionskern (230), der zwischen der Oberschicht (210) und der Unterschicht (220) angeordnet ist, und
ein Paar Sperrbündchen (300), die an der Absorptionseinheit (200) angeordnet sind und sich in Längsrichtung erstrecken, wobei jedes Sperrbündchen (300) Folgendes aufweist:
einen festen Rand (330), der an der Absorptionseinheit (200) befestigt ist,
einen freien Rand (340) gegenüber dem festen Rand (330),
einen ersten gefalteten Abschnitt (350), der zwischen dem festen Rand (330) und dem freien Rand (340) gebildet wird, indem das Sperrbündchen (300) an beiden Längsenden (301, 302) des Sperrbündchens (300) quer nach außen gefaltet wird, und
einen zweiten gefalteten Abschnitt (360), der gebildet wird, indem das Sperrbündchen (300) an beiden Längsenden (301, 302) des Sperrbündchens (300) quer nach außen gefaltet wird, so dass der zweite gefaltete Abschnitt (360) an jedem Längsende (301, 302) des Sperrbündchens (300) quer nach innen von dem ersten gefalteten Abschnitt (350) und dem freien Rand (340) aus angeordnet ist,
wobei jedes Sperrbündchen (300) mit mindestens zwei elastischen Elementen (321, 322) versehen ist, so dass mindestens ein elastisches Element (321) entlang des freien Randes (340) angeordnet ist und so dass mindestens ein elastisches Element (322) entlang des ersten gefalteten Abschnitts (350) angeordnet ist.

2. Absorptionsartikel (100) nach Anspruch 1, wobei das Sperrbündchen (300) an den Längsenden (301, 302) des Sperrbündchens (300) quer nach außen gefaltet ist, so dass der erste gefaltete Abschnitt (350) an jedem Längsende (301, 302) des Sperrbündchens (300) mit dem freien Rand (340) fluchtet.

3. Absorptionsartikel (100) nach Anspruch 1, wobei das Sperrbündchen (300) an den Längsenden (301, 302) des Sperrbündchens (300) quer nach außen gefaltet ist, so dass der erste gefaltete Abschnitt (350) an jedem Längsende (301, 302) des Sperrbündchens (300) quer nach außen von dem freien Rand (340) aus angeordnet ist.

4. Absorptionsartikel (100) nach Anspruch 1, wobei das Sperrbündchen (300) an den Längsenden (301, 302) des Sperrbündchens (300) quer nach außen gefaltet ist, so dass der erste gefaltete Abschnitt (350) an jedem Längsende (301, 302) des Sperrbündchens (300) quer nach innen von dem freien Rand (340) aus angeordnet ist.

5. Absorptionsartikel (100) nach Anspruch 1, wobei der freie Rand (340) eine längere elastifizierte Länge besitzt als der erste gefaltete Abschnitt (350).

6. Absorptionsartikel (100) nach Anspruch 5, wobei der freie Rand (340) eine elastifizierte Länge von etwa 65 % bis etwa 80 % der longitudinalen Gesamtlänge des Absorptionsartikels (100) bestimmt, während der erste gefaltete Abschnitt (350) eine elastifizierte Länge von etwa 40 % bis etwa 60 % der longitudinalen Gesamtlänge des Absorptionsartikels (100) bestimmt.

7. Absorptionsartikel (100) nach Anspruch 1, wobei der erste gefaltete Abschnitt (350) eine längere elastifizierte Länge besitzt als der freie Rand (340).

8. Absorptionsartikel (100) nach Anspruch 7, wobei der erste gefaltete Abschnitt (350) eine elastifizierte Länge von etwa 80 % bis etwa 95 % der longitudinalen Gesamtlänge des Absorptionsartikels (100) bestimmt, während der freie Rand (340) eine elastifizierte Länge von etwa 50 % bis etwa 75 % der longitudinalen Gesamtlänge des Absorptionsartikels (100) bestimmt.

## Revendications

1. Article absorbant (100) possédant une ligne médiane longitudinale (L) et une ligne médiane transversale (T), l'article absorbant (100) comprenant :
un ensemble absorbant (200) comprenant une feuille de dessus perméable aux liquides (210), une feuille de fond imperméable aux liquides (220) et une âme absorbante (230) positionnée entre la feuille de dessus (210) et la feuille de fond (220) ; et
une paire de revers formant barrière (300) disposés sur l'ensemble absorbant (200) et s'étendant longitudinalement, chaque revers formant barrière (300) possédant :
un bord fixe (330) fixé à l'ensemble absorbant (200),
un bord libre (340) opposé au bord fixe (330),
une première partie pliée (350) formée entre le bord fixe (330) et le bord libre (340) par pliage du revers formant barrière (300) transversalement vers l'extérieur au niveau de l'une et l'autre des extrémités longitudinales (301, 302) du revers formant barrière (300), et
une deuxième partie pliée (360) formée par pliage du revers formant barrière (300) transversalement vers l'extérieur au niveau de l'une et l'autre des extrémités longitudinales (301, 302) du revers formant barrière (300) de sorte que la deuxième partie pliée (360) est positionnée transversalement vers l'intérieur à partir de la première partie pliée (350) et du bord libre (340) au niveau de chaque extrémité longitudinale (301, 302) du revers formant barrière (300),
chaque revers formant barrière (300) étant pourvu d'au moins deux éléments élastiques (321, 322) de sorte qu'au moins un élément élastique (321) est positionné le long du bord libre (340) et de sorte qu'au moins un élément élastique (322) est positionné le long de la première partie pliée (350).

2. Article absorbant (100) selon la revendication 1, dans lequel le revers formant barrière (300) est plié transversalement vers l'extérieur au niveau des extrémités longitudinales (301, 302) du revers formant barrière (300) de sorte que la première partie pliée (350) est alignée avec le bord libre (340) au niveau de chaque extrémité longitudinale (301, 302) du revers formant barrière (300).

3. Article absorbant (100) selon la revendication 1, dans lequel le revers formant barrière (300) est plié transversalement vers l'extérieur au niveau des extrémités longitudinales (301, 302) du revers formant barrière (300) de sorte que la première partie pliée (350) est positionnée transversalement vers l'extérieur à partir du bord libre (340) au niveau de chaque extrémité longitudinale (301, 302) du revers formant barrière (300).

4. Article absorbant (100) selon la revendication 1, dans lequel le revers formant barrière (300) est plié transversalement vers l'extérieur au niveau des extrémités longitudinales (301, 302) du revers formant barrière (300) de sorte que la première partie pliée (350) est positionnée transversalement vers l'intérieur à partir du bord libre (340) au niveau de chaque extrémité longitudinale (301, 302) du revers formant barrière (300).

5. Article absorbant (100) selon la revendication 1, dans lequel le bord libre (340) a une longueur élastifiée plus longue que la première partie pliée (350).

6. Article absorbant (100) selon la revendication 5, dans lequel le bord libre (340) définit une longueur élastifiée allant d'environ 65 % à environ 80 % de la longueur longitudinale globale de l'article absorbant (100) tandis que la première partie pliée (350) définit une longueur élastifiée allant d'environ 40 % à environ 60 % de la longueur longitudinale globale de l'article absorbant (100).

7. Article absorbant (100) selon la revendication 1, dans lequel la première partie pliée (350) a une longueur élastifiée plus longue que le bord libre (340).

8. Article absorbant (100) selon la revendication 7, dans lequel la première partie pliée (350) définit une longueur élastifiée allant d'environ 80 % à environ 95 % de la longueur longitudinale globale de l'article absorbant (100) tandis que le bord libre (340) définit une longueur élastifiée allant d'environ 50 % à environ 75 % de la longueur longitudinale globale de l'article absorbant (100).
